# EUROPÄISCHE PATENTSCHRIFT

(11) **EP 0 508 330 B1**
(45) Veröffentlichungstag und Bekanntmachung des Hinweises auf die Patenterteilung: **16.08.1995**
(21) Anmeldenummer: 92105821.0
(22) Anmeldetag: 03.04.1992
(51) Int. Cl.: A61K 35/78

(54) **Verfahren zur Herstellung von Extrakten aus Rhizoma Petasitidis mit Kohlendioxid**
Rhizoma petasitidis extraction process with carbon dioxide
Procédé d'extraction du rhizoma petasitidis à l'aide de dioxyde de carbone

(30) Priorität: 06.04.1991 DE 4111141
(43) Veröffentlichungstag der Anmeldung: 14.10.1992
(73) Patentinhaber: SKW Trostberg Aktiengesellschaft, 83308 Trostberg (DE)
(72) Erfinder: Zobel, Rudolf, I-86074 Filignano (IT); Simon, Andrea, W-8221 Seebruck (DE)
(74) Vertreter: Böhm, Brigitte, Dipl.-Chem. Dr.

(56) Entgegenhaltungen:
- EP-A- 0 391 504
- DE-A- 4 002 938
- CHEMICAL ABSTRACTS, vol. 104, no. 4, 27 January 1986, Columbus, Ohio, US;abstract no. 39564C, MAUZ C. ET AL: 'Methods for the removal of pyrrolizidine
- alkaloids from medicinal plant extracts.' page 369 ;column 1 ;

## Beschreibung

Gegenstand der vorliegenden Erfindung ist ein Verfahren zur Herstellung von Extrakten aus Rhizoma Petasitidis mit einem sehr geringen Gehalt an Pyrrolizidin-Alkaloiden.

Wegen ihrer schleimlösenden Wirkung bei Husten und Asthma sowie wegen ihrer spasmolytischen Wirkung bei Krampfzuständen wurde die Pestwurz (Petasites hybridus oder Petasites officinalis) bereits in der Volksheilkunde verwendet. Als Hauptwirkstoff der aus den Wurzeln der Pestwurz (Rhizoma Petasitidis) extrahierbaren Substanzen ist das Petasin anzusehen, ein Ester aus Petasol und Angelicasäure. Daneben sind in dieser Arzneimittelpflanze eine Reihe von Pyrrolizidin-Alkaloiden nachgewiesen worden wie z. B. das Hauptalkaloid Senecionin. Das hepatotoxische und kanzerogene Potential dieser Pyrrolizidin-Alkaloide hat in der Öffentlichkeit kontrovers geführte Diskussionen über die Verwendung dieses Extraktes als Arzneimittel ausgelöst. In jedem Fall ist es aus Gründen der Vorsorge wünschenswert, den Gehalt an Pyrrolizidin-Alkaloiden in Extrakten aus Rhizoma Petasitidis so gering wie möglich zu halten bzw. mit Hilfe geeigneter Verfahren unter die Nachweisgrenze abzusenken.

Zur Lösung dieses Problems sind bisher zwei Verfahren bekannt geworden. Gemäß dem erstgenannten Verfahren (vgl. Ch. Mauz et al. in Pharmaceutica Acta Helvetiae Band 60 (1985) S. 256 - 59) wird ein alkoholischer Extrakt aus Rhizoma Petasitidis mit einem Kationenaustauscher behandelt. Auf diese Weise werden mehr als 93 % der Pyrrolizidin-Alkaloide am Ionenaustauscher adsorbiert. Dieses Verfahren ist ziemlich aufwendig, weil der Extrakt anschließend von Ionenaustauscher, Alkohol und Wasser wieder abgetrennt werden muß, was im Falle von Alkohol und Wasser wegen der Temperaturempfindlichkeit der Pflanzeninhaltsstoffe nicht unproblematisch ist. Ferner besteht bei Verwendung von Ionenaustauschern die Möglichkeit der Entstehung von Substanzen, die im Extrakt vor der Behandlung nicht vorhanden waren.

Das in der DE-PS 39 10 831 (& EP-A-0 391 504) beschriebene Verfahren nutzt die guten Lösungseigenschaften von verdichtetem Kohlendioxid für Petasine aus dem Rhizom der Pestwurz. Die Pyrrolizidin-Alkaloide werden durch das Extraktionsmittel CO₂ in wesentlich geringerem Umfang gelöst, so daß man Extrakte erhält, deren Gehalt an Pyrrolizidin-Alkaloiden unter 5 ppm liegt. Ein sicheres Absenken der Pyrrolizidin-Alkaloide unter die Nachweisgrenze von 0,1 ppm ist nach diesem Verfahren bisher nicht möglich.

Der vorliegenden Erfindung lag daher die Aufgabe zugrunde, ein Verfahren zur Herstellung von Extrakten aus Rhizoma Petasitidis mit einem sehr geringen Gehalt an Pyrrolizidin-Alkaloiden durch Extraktion der Droge mit verdichtetem Kohlendioxid bei > 8 MPa und anschließender Abtrennung des Extraktes vom Lösemittel durch Dichteerniedrigung zu entwickeln, welches die genannten Nachteile des Standes der Technik nicht aufweist, sondern mit geringem technischen Aufwand eine möglichst hohe Ausbeute an pharmazeutisch wirksamen Pestwurz-Inhaltsstoffen ermöglicht.

Diese Aufgabe wurde erfindungsgemäß dadurch gelöst, daß man den Extrakt nach der Abtrennung des Lösemittels mit der ein- bis zehnfachen Menge an Wasser behandelt und den Extrakt nach dessen Abtrennung von der wäßrigen Phase trocknet. Es hat sich nämlich überraschenderweise gezeigt, daß man mit Hilfe des erfindungsgemäßen Verfahrens den Hauptwirkstoff der Pestwurzdroge, das Petasin, in sehr guten Ausbeuten gewinnen und gleichzeitig den Gehalt an unerwünschten Pyrrolizidin-Alkaloiden unter die Nachweisgrenze von 0,1 ppm reduzieren kann.

Beim Verfahren entsprechend der vorliegenden Erfindung wird die Pestwurzdroge, die in der Regel einen Gesamtalkaloidgehalt in der Größenordnung von 1 - 50 mg pro kg Trockengewicht und einen Wassergehalt von 8 - 12 Gew.-% aufweist, in zerkleinerter Form eingesetzt. Das Zerkleinern von Rhizoma Petasitidis kann nach den üblichen Methoden, wie z. B. Mahlen, und mit bekannten technischen Vorrichtungen erfolgen.

Die Extraktionsbedingungen können insbesondere in Bezug auf den Druck in weiten Grenzen variiert werden, wobei die Untergrenze für die Extraktion mit CO₂ ca. 8 MPa beträgt. Gemäß einer bevorzugten Ausführungsform wird in einem Druckbereich von 10 - 30 MPa, insbesondere 13 - 28 MPa, extrahiert. Die Temperatur kann über oder unter der kritischen Temperatur des CO₂ (31°C) liegen. Wegen der Temperaturempfindlichkeit der Drogeninhaltsstoffe sollten aber 45°C nicht wesentlich überschritten werden. Vorzugsweise wird die Temperatur während der Extraktion auf 25 - 45°C, insbesondere 32 - 40°C, eingestellt.

Das Verhältnis von CO₂-Gasmenge zur Menge der eingesetzten Pestwurzdroge hat sowohl Einfluß auf die Ausbeute an Extrakt als auch auf den Gehalt an Pyrrolizidin-Alkaloiden im Extrakt. Vorzugsweise wird mit 5 - 50 kg CO₂ pro kg Droge extrahiert. Nach der Extraktion werden die gelösten Pestwurz-Inhaltsstoffe in einer Abscheidestufe vom Lösemittel CO₂ durch Dichteerniedrigung abgetrennt. Die Abtrennung vom Lösemittel wird vorzugsweise bei einem Druck von 3 - 5 MPa und einer Temperatur von 10 - 30°C vorgenommen, wobei ein Pestwurz-Extrakt mit einem Wassergehalt von ca. 20 Gew.-% anfällt. Das überstehende Wasser kann beispielsweise durch Dekantieren abgetrennt werden.

Es ist erfindungswesentlich, daß man den Pestwurz-Extrakt, der noch merkliche Mengen (bis 5 ppm) an Pyrrolizidin-Alkaloiden enthalten kann, einer Behandlung mit Wasser unterwirft. Zur Entfernung dieser Restmengen an Pyrrolizidin-Alkaloiden wird der Pestwurz-Extrakt mit der ein- bis zehnfachen, vorzugsweise der doppelten bis fünffachen Menge an Wasser versetzt und intensiv vermischt. Gemäß einer bevorzugten Ausführungsform kann das Wasser zur Behandlung des Pestwurz-Extraktes auch leicht angesäuert sein und bspw. einen pH-Wert von 3 bis 7 aufweisen. Die Wasserbehandlung kann sowohl bei Raumtemperatur als auch bei leicht erhöhter Temperatur stattfinden, wobei Temperaturen von 20 - 50°C, insbesondere 35 - 45°C, als bevorzugt anzusehen sind. Je nach eingesetzter Wassermenge und Behandlungstemperatur sind in der Regel Behandlungszeiten von 1 - 30 Stunden ausreichend.

Anschließend kann bspw. nach mehrstündigem Ruhenlassen der Extrakt-Wasser-Mischung zur Phasentrennung ein Großteil der wäßrigen Phase mit den darin gelösten Alkaloiden durch Dekantieren von der lipophilen Extraktphase abgetrennt werden. Der Pestwurz-Extrakt enthält nach diesem Verfahrensschritt noch ca. 10 - 20 Gew.-% emulgiertes Wasser.

Abschließend wird dem noch milchig-trüben Pestwurz-Extrakt durch Trocknung Wasser entzogen, bis man einen braun-gelben, klaren Extrakt von honigartiger Konsistenz erhält. Dazu wird der wasserhaltige Extrakt bspw. durch Filtration oder Zentrifugation über ein Trockenmittel, vorzugsweise Natriumsulfat, behandelt, bis ein klares, direkt verkaufsfähiges Produkt mit einem Wassergehalt von weniger als 1 Gew.-% entsteht.

Mit Hilfe des erfindungsgemäßen Verfahrens ist es möglich, einen hochwertigen Pestwurz-Extrakt herzustellen, der aus bis zu 40 Gew.-% Petasin besteht und einen Gehalt an Pyrrolizidin-Alkaloiden von weniger als 0,1 ppm aufweist. Bei einem durchschnittlichen Alkaloidgehalt in der Droge von 30 ppm und einer Extraktausbeute von 2 - 3 Gew.-% sind in einem solchen Extrakt maximal noch 0,01 % der ursprünglich vorhandenen Alkaloide enthalten, während der Petasin-Wirkstoff mit einer Ausbeute von mindestens 90 % erhalten wird.

Die nachfolgenden Beispiele sollen die Erfindung näher erläutern.

### Beispiel 1

60 kg zerkleinerte Droge (mittlere Korngröße 740 »m) aus dem Petasites-Rhizom mit einem Petasingehalt von ca. 1 Gew.-% und einem Gehalt an Pyrrolizidin-Alkaloiden von 20 bis 30 ppm sowie einer Feuchte von 9,5 Gew.-% wurden mit überkritischem Kohlendioxid bei einem Druck von 26 MPa und einer Temperatur von 35°C extrahiert. Je kg Droge wurden 9,3 kg CO₂ eingesetzt. Die Extraktabscheidung aus dem Lösemittel Kohlendioxid erfolgte kontinuierlich durch Dichteerniedrigung (4,5 MPa/30°C). Das regenerierte Kohlendioxid wurde erneut in den Extraktionsautoklaven zurückgeführt. Als Primär-Extrakt wurden 2,6 kg (4 Gew.-%) einer trüben Extrakt-Wasser-Mischung mit ca. 20 bis 25 Gew.-% Wasser erhalten. Nach einigen Stunden konnte ein Teil der wäßrigen Phase mechanisch abgetrennt werden. Eine Untersuchung dieses Extraktes auf Pyrrolizidin-Alkaloide ergab eine Konzentration von 1 ppm. 500 g des Extraktes wurden mit 1000 g bidestilliertem Wasser intensiv vermischt und bei 45°C 24 Stunden lang in Bewegung gehalten. Nach Entmischung wurden ca. 950 g der wäßrigen Phase von der Extraktphase abgezogen. Eine kleine Menge des Extraktes wurde mit der gleichen Menge wasserfreiem Natriumsulfat zu einem Brei angerührt und in einer Laborzentrifuge kurz angeschleudert. Die restliche Extraktmenge wurde kontinuierlich in die Zentrifuge eingefüllt und über die Natriumsulfatschicht getrocknet. Ergebnis waren 395 g eines klaren, hochviskosen Pestwurz-Extraktes mit einem Petasingehalt von ca. 40 Gew.-% und einem Wassergehalt von 0,5 Gew.-% und einem nicht mehr nachweisbaren Gehalt an Pyrrolizidin-Alkaloiden (Nachweisgrenze 0,1 ppm).

### Beispiel 2 (Vergleich)

2 kg einer zerkleinerten Pestwurz-Droge aus dem Petasites-Rhizom mit einer Feuchte von 10 Gew.-%, einem Petasingehalt von ca. 1 Gew.-% und einem Pyrrolizidin-Alkaloid-Gehalt von ca. 20 bis 30 ppm wurden mit CO₂ bei einem Druck von 16 MPa und einer Temperatur von 32°C extrahiert. Der CO₂-Durchsatz betrug 20 kg CO₂ pro Stunde. Die Extraktabscheidung wurde bei einem Druck von 40 bar und einer Temperatur von 22°C vorgenommen. Nach einer Stunde wurde die Extraktion beendet. Es fielen 51 g Extrakt mit einem Petasingehalt von ca. 40 Gew.-% und einem Wassergehalt von 0,58 Gew.-% an, dessen Pyrrolizidin-Gehalt noch 0,7 ppm betrug.

## Patentansprüche

1. Verfahren zur Herstellung von Extrakten aus Rhizoma Petasitidis mit einem sehr geringen Gehalt an Pyrrolizidin-Alkaloiden durch Extraktion der Droge mit verdichtetem Kohlendioxid bei > 8 MPa und anschließender Abtrennung des Extraktes vom Lösemittel durch Dichteerniedrigung, dadurch gekennzeichnet, daß man den Extrakt nach der Abtrennung des Lösemittels mit der ein- bis zehnfachen Menge an Wasser behandelt und den Extrakt nach dessen Abtrennung von der wäßrigen Phase trocknet.

2. Verfahren nach Anspruch 1, dadurch gekennzeichnet, daß man die Extraktion bei einem Druck von 10 - 30 MPa, vorzugsweise 13 - 28 MPa, durchführt.

3. Verfahren nach den Ansprüchen 1 und 2, dadurch gekennzeichnet, daß man die Extraktionstemperatur auf 25 - 45°C, vorzugsweise 32 - 40°C, einstellt.

4. Verfahren nach den Ansprüchen 1 bis 3, dadurch gekennzeichnet, daß der CO₂-Durchsatz 5 - 50 kg CO₂ pro kg Ausgangsmaterial beträgt.

5. Verfahren nach den Ansprüchen 1 bis 4, dadurch gekennzeichnet, daß man die Abtrennung vom Lösemittel bei einem Druck von 3 bis 5 MPa und einer Temperatur von 10 - 30°C vornimmt.

6. Verfahren nach den Ansprüchen 1 bis 5, dadurch gekennzeichnet, daß man den Extrakt nach der Abtrennung des Lösemittels mit der doppelten bis fünffachen Menge an Wasser behandelt.

7. Verfahren nach Anspruch 6, dadurch gekennzeichnet, daß man die Behandlung mit Wasser bei einer Temperatur von 20 - 50°C, vorzugsweise bei einer Temperatur von 35 - 45°C, vornimmt.

8. Verfahren nach den Ansprüchen 1 bis 7, dadurch gekennzeichnet, daß man die Trocknung des Extraktes durch Filtration oder Zentrifugation mit Hilfe eines Trockenmittels, vorzugsweise Natriumsulfat, vornimmt.

9. Verfahren nach den Ansprüchen 1 bis 8, dadurch gekennzeichnet, daß man die Trocknung des Extraktes bis zu einem Wassergehalt < 1 % durchführt.

## Claims

1. Process for the preparation of extracts from petasitidis rhizomes with a very low content of pyrrolizidine alkaloids by extraction of the drug with compressed carbon dioxide at >8 MPa and subsequent separation of the extract from the solvent by density lowering, characterised in that, after the separation of the solvent, one treats the extract with a one to ten fold amount of water and dries the extract after its separation from the aqueous phase.

2. Process according to claim 1, characterised in that one carries out the extraction at a pressure of 10 - 30 MPa, preferably of 13 - 28 MPa.

3. Process according to claims 1 and 2, characterised in that one adjusts the extraction temperature to 25 - 45°C, preferably to 32 - 40°C.

4. Process according to claims 1 to 3, characterised in that the CO₂ throughput amounts to 5 - 50 kg of CO₂ per kg of starting material.

5. Process according to claims 1 to 4, characterised in that one carries out the separation of the solvent at a pressure of 3 to 5 MPa and at a temperature of 10 - 30°C.

6. Process according to claims 1 to 5, characterised in that, after the separation of the solvent, one treats the extract with the double to five fold amount of water.

7. Process according to claim 6, characterised in that one carries out the treatment with water at a temperature of 20 - 50°C, preferably at a temperature of 35 - 45°C.

8. Process according to claims 1 to 7, characterised in that one carries out the drying of the extract by filtering or centrifuging with the help of a drying agent, preferably sodium sulphate.

9. Process according to claims 1 to 8, characterised in that one carries out the drying of the extract up to a water content of < 1%.

## Revendications

1. Procédé pour la production d'extraits à partir de rhizoma petasitidis ayant une très faible teneur en alcaloïdes de pyrrolizidine par extraction du produit médicinal avec du dioxyde de carbone comprimé à > 8 MPa et séparation consécutive de l'extrait du solvant par réduction de la densité, caractérisé en ce que l'on traite l'extrait après la séparation du solvant avec une quantité égale à 1 jusqu'à 10 fois en eau et on sèche l'extrait après sa séparation de la phase aqueuse.

2. Procédé selon la revendication 1, caractérisé en ce que l'on effectue l'extraction à une pression de 10 - 30 MPa, de préférence 13 - 28 MPa.

3. Procédé selon les revendications 1 et 2, caractérisé en ce que l'on règle la température d'extraction à 25 - 45°C, de préférence 32 - 40°C.

4. Procédé selon les revendications 1 à 3, caractérisé en ce que le débit en CO₂ est de 5 - 50 k CO₂ par kg de matière de départ.

5. Procédé selon les revendications 1 à 4, caractérisé en ce que l'on procède à la séparation du solvant à une pression de 3 à 5 MPa et à une température de 10 - 30°C.

6. Procédé selon les revendications 1 à 5, caractérisé en ce que l'on traite l'extrait après la séparation du solvant avec le double ou le quintuple de la quantité en eau.

7. Procédé selon la revendication 6, caractérisé en ce que l'on procède au traitement à l'eau à une température de 20 à 50°C, de préférence à une température de 35 à 45°C.

8. Procédé selon les revendications 1 à 7, caractérisé en ce que l'on procède au séchage de l'extrait par filtration ou centrifugation à l'aide d'un agent de séchage, de préférence du sulfate de sodium.

9. Procédé selon les revendications 1 à 8, caractérisé en ce que l'on procède au séchage de l'extrait jusqu'à une teneur en eau < 1 %.
